# NOUVEAU FASCICULE DE BREVET EUROPEEN

(11) **EP 1 437 123 B2**
(45) Date de publication et mention de la décision concernant l'opposition: **08.11.2017**
(45) Mention de la délivrance du brevet: 06.06.2007
(21) Numéro de dépôt: 04290396.3
(22) Date de dépôt: 07.10.1998
(51) Int. Cl.: A61K 8/41, A61Q 5/10, A61Q 5/06, A61K 8/49

(54) **COMPOSITION DE TEINTURE DES FIBRES KÉRATINIQUES ET PROCÉDÉ DE TEINTURE METTANT EN OEUVRE CETTE COMPOSITION**
FÄRBEMITTEL FÜR KERATINISCHE FASER UND VERFAHREN ZUM FÄRBEN UNTER VERWENDUNG DIESER ZUSAMMENSETZUNG
COMPOSITION FOR DYEING KERATIN FIBRES AND DYEING METHOD USING SAME

(30) Priorité: 22.10.1997 FR 9713240
(43) Date de publication de la demande: 14.07.2004
(62) Demande divisionnaire de: 98947623.9
(73) Titulaire: L'Oréal, 75008 Paris (FR)
(72) Inventeur: Rondeau, Christine, 78500 Sartrouville (FR)
(74) Mandataire: Kuhlmann, Sonia

(56) Documents cités:
- WO-A-95/01772
- US-A- 3 985 499
- US-A- 4 025 301

## Description

L'invention a pour objet une composition pour la teinture des fibres kératiniques, et en particulier des fibres kératiniques humaines telles que les cheveux comprenant, dans un milieu approprié pour la teinture, au moins un colorant direct cationique convenablement sélectionné, et au moins un colorant direct nitré benzénique, ainsi que le procédé de teinture mettant en oeuvre cette composition.

Il est bien connu de teindre les fibres kératiniques et en particulier les cheveux humains avec des compositions tinctoriales contenant des colorants directs et en particulier des colorants directs nitrés benzéniques. D'autres colorants connus dans le domaine sont des colorants directs cationiques diazamerocyanines décrits dans le brevet US 3985499 Les colorants directs ont cependant l'inconvénient, lorsqu'ils sont incorporés dans des compositions tinctoriales, de conduire à des colorations présentant une ténacité insuffisante, en particulier vis-à-vis des shampooings.

Or, la demanderesse vient maintenant de découvrir qu'il est possible d'obtenir de nouvelles teintures capables de conduire à des colorations puissantes, peu sélectives et résistant bien aux diverses agressions que peuvent subir les cheveux, en associant au moins un colorant direct cationique convenablement sélectionné, et au moins un colorant direct nitré benzénique.

Cette découverte est à la base de la présente invention.

L'invention a donc pour premier objet une composition prête à l'emploi, pour la teinture des fibres kératiniques et en particulier des fibres kératiniques humaines telles que les cheveux, caractérisée par le fait qu'elle comprend, dans un milieu approprié pour la teinture :
- au moins un colorant direct cationique choisi parmi :
   b) les composés de formules (III) suivantes : 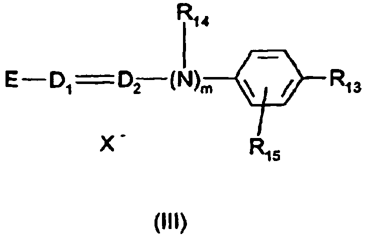 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      D₁ et D₂, identiques ou différents, représentent un atome d'azote ou le groupement -CH,
      m = 1,
      X⁻ représente un anion de préférence choisi parmi le chlorure, le méthyl sulfate et l'acétate,
      E représente un groupement choisi par les structures suivantes : 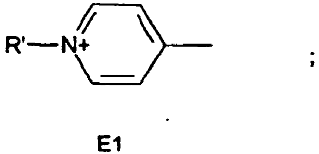
      dans lesquelles R' représente un radical alkyle en C₁-C₄ ;
- et au moins un colorant direct nitré benzénique.

La composition tinctoriale prête à l'emploi conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture des fibres kératiniques mettant en oeuvre cette composition tinctoriale prête à l'emploi.

Les colorants directs cationiques de formules (I), (II), (III) et (III') utilisables dans les compositions tinctoriales prêtes à l'emploi conformes à l'invention, sont des composés connus et sont décrits par exemple dans les demandes de brevets WO 95/01772, WO 95/15144 et EP-A-0 714 954.

Parmi les colorants directs cationiques de formule (III), utilisables dans les compositions tinctoriales prêtes à l'emploi conformes à l'invention, on peut plus particulièrement citer les composés répondant aux structures suivantes :

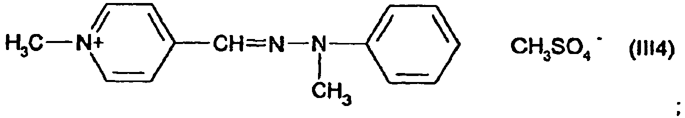

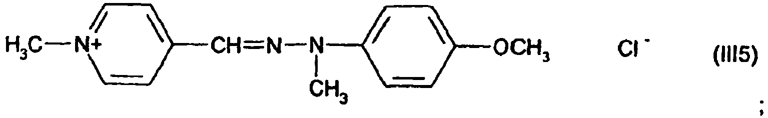

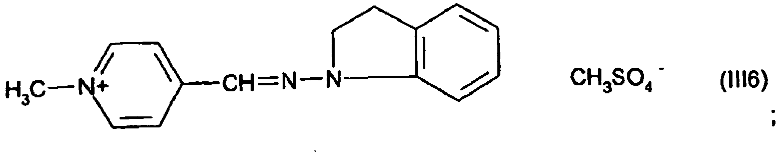

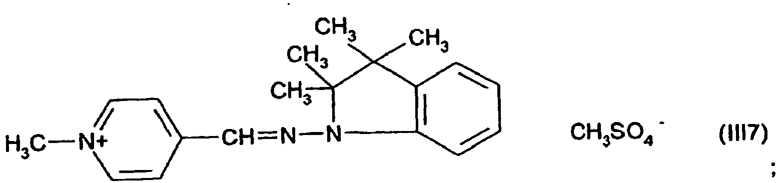

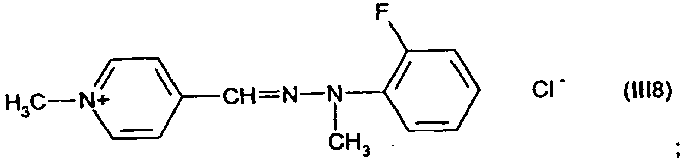

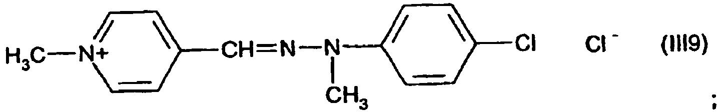

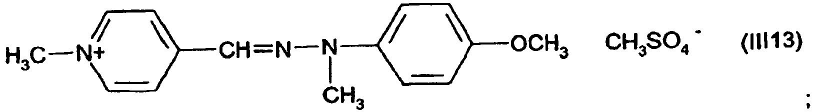

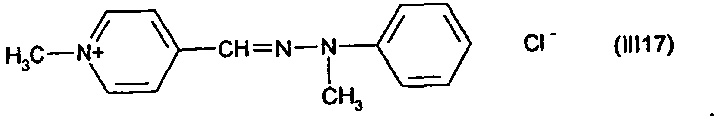

Parmi les composés particuliers de structures décrits ci-dessus, on préfère tout particulièrement les composés répondant aux structures (III4), (III5) et (III13).

Le ou les colorants directs cationiques utilisés selon l'invention, représentent de préférence de 0,001 à 10 % en poids environ du poids total de la composition tinctoriale prête à l'emploi et encore plus préférentiellement de 0,05 à 5 % en poids environ de ce poids.

Le ou les colorants directs nitrés benzéniques pouvant être utilisés dans la composition tinctoriale prête à l'emploi conforme à l'invention sont de préférence choisis parmi les composés de formule (IV) suivante : 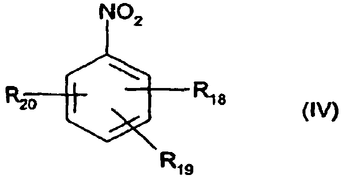 dans laquelle:
- R₁₈ représente un radical amino ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino,
- R₁₉ représente un atome d'hydrogène ; un radical amino ; hydroxyle ; alkyle en C₁-C₄ ; alcoxy en C₁-C₄ ; monohydroxyalkyle en C₁-C₄ ; polyhydroxyalkyle en C₂-C₄ ; monohydroxyalcoxy en C₁-C₄ ; polyhydroxyalcoxy en C₂-C₄ ; aminoalcoxy en C₁-C₄ ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino ;
- R₂₀ représente un atome d'hydrogène ou d'halogène, un radical alkyle en C₁-C₄, ou un groupement nitro.

Parmi les colorants nitrés benzéniques de formule (IV) ci-dessus, on peut tout particulièrement citer :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- le 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyéthyl)amino 5-méthoxy nitrobenzène,
- le 2-amino 5-β-hydroxyéthyloxy nitrobenzène,
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β,γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-β,γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthyl)amino nitrobenzène,
- le 2-amino 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino 5-amino nitrobenzène,
- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-(β-aminoéthyl)amino 4-hydroxy nitrobenzène,
- l'[hydroxy-2 N-(β-hydroxyéthyl)amino-3 nitro-6] benzyloxy]-2 éthylamine, et
- l'[hydroxy-2 N-(β-hydroxypropyl)amino-3 nitro-6] benzyloxy]-2 éthylamine.

Parmi les colorants nitrés benzéniques de formule (IV) ci-dessus, on préfère tout particulièrement :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

Le ou les colorants nitrés benzéniques représentent de préférence de 0,0005 à 15 % en poids environ du poids total de la composition tinctoriale prête à l'emploi conforme à l'invention, et encore plus préférentiellement de 0,005 à 10 % en poids environ de ce poids.

La composition tinctoriale prête à l'emploi conforme à l'invention peut en outre renfermer une ou plusieurs bases d'oxydation et/ou un ou plusieurs coupleurs. Ces bases d'oxydation peuvent notamment être choisies parmi les paraphénylènediamines, les para-aminophénols, les orthophénylènediamines et les bases hétérocycliques telles que par exemple les dérivés pyridiniques, les dérivés pyrimidiniques, les dérivés pyrazoliques, et les dérivés pyrazolo-pyrimidiniques. Les coupleurs peuvent notamment être choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Lorsqu'elles sont présentes, la ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids.

Lorsqu'ils sont présents, le ou les coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de invention (bases d'oxydation et coupleurs) sont notamment choisis parmi les chlorhydrates, les bromhydrates, les sulfates, les tartrates, les lactates et les acétates.

Lorsqu'une ou plusieurs bases d'oxydation et/ou un ou plusieurs coupleurs sont utilisés, alors la composition tinctoriale prête à l'emploi peut en outre renfermer au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux électrons.

Parmi les oxydo-réductases à 2 électrons pouvant être utilisées à titre d'agent oxydant dans la composition tinctoriale prête à l'emploi conforme à l'invention, on peut plus particulièrement citer les pyranose oxydases, les glucose oxydases, les glycérol oxydases, les lactates oxydases, les pyruvate oxydases, et les uricases.

Selon l'invention, l'utilisation des uricases d'origine animale, microbiologique ou biotechnologique est particulièrement préférée.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

Lorsqu'elles sont utilisées, la ou les oxydo-réductases à 2 électrons représentent de préférence de 0,01 à 20 % en poids environ du poids total de la composition tinctoriale prête à l'emploi, et encore plus préférentiellement de 0,1 à 5 % en poids environ de ce poids.

Lorsqu'une enzyme de type oxydo-réductase à 2 électrons est utilisée conformément à l'invention, la composition tinctoriale prête à l'emploi peut en outre renfermer un ou plusieurs donneurs pour ladite enzyme.

Selon l'invention, on entend par donneur, les différents substrats participant au fonctionnement de ladite ou desdites oxydo-réductases à 2 électrons.

La nature du donneur (ou substrat) utilisé varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Lorsqu'ils sont utilisés, le ou les donneurs (ou substrats) représentent de préférence de 0,01 à 20 % en poids environ du poids total de la composition tinctoriale prête à l'emploi conforme à l'invention et encore plus préférentiellement de 0,1 à 5 % en environ de ce poids.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique ou le phénoxyéthanol, les produits analogues et leurs mélanges.

Le pH de la composition prête à l'emploi conforme à l'invention est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (V) suivante : 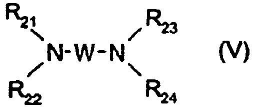 dans laquelle W est un reste propylène éventuellement substitué par un groupement hydroxyle ou un radical alkyle en C₁-C₄ ; R₂₁, R₂₂, R₂₃ et R₂₄, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que par exemple des agents antioxydants, des agents de pénétration, des agents séquestrants, des parfums, des tampons, des agents dispersants, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

Lorsque la composition tinctoriale prête à l'emploi conforme à l'invention renferme au moins une base d'oxydation et/ou au moins un coupleur et au moins un agent oxydant, elle doit alors être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, et lorsque la composition tinctoriale conforme à l'invention renferme au moins une base d'oxydation et/ou au moins un coupleur, le procédé comporte une étape préliminaire consistant à stocker sous forme séparée, d'une part, une composition (A) comprenant, dans un milieu approprié pour la teinture, au moins un colorant direct cationique tel que défini précédemment, au moins un colorant direct nitré benzénique et au moins une base d'oxydation et/ou au moins un coupleur et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## Revendications

1. Composition prête à l'emploi, pour la teinture des fibres kératiniques et en particulier des fibres kératiniques humaines telles que les cheveux, **caractérisée par le fait qu'**elle comprend, dans un milieu approprié pour la teinture :
- au moins un colorant direct cationique choisi parmi :
b) les composés de formules (III) suivantes : 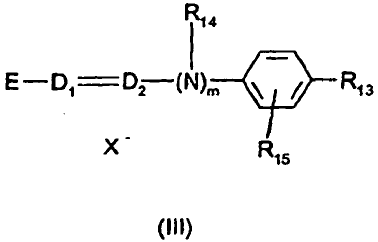 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
D₁ et D₂, identiques ou différents, représentent un atome d'azote ou le groupement -CH,
m = 1,
X⁻ représente un anion de préférence choisi parmi le chlorure, le méthyl sulfate et l'acétate,
E représente un groupement choisi par les structures suivantes : 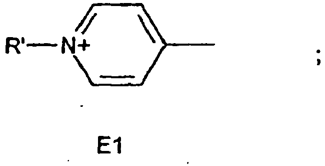
dans lesquelles R' représente un radical alkyle en C₁-C₄;
- et au moins un colorant direct nitré benzénique.

2. Composition selon la revendication 1, **caractérisée par le fait que** les colorants directs cationiques de formule (III) sont choisis parmi les composés répondant aux structures suivantes :

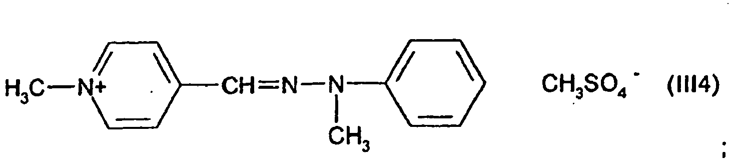

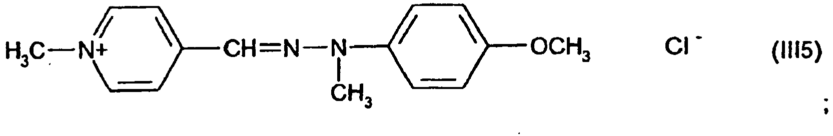

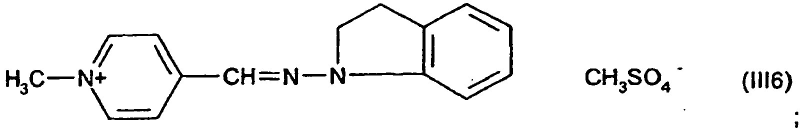

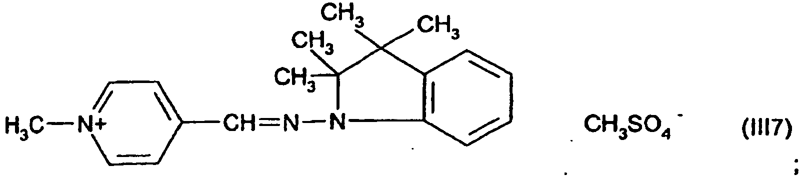

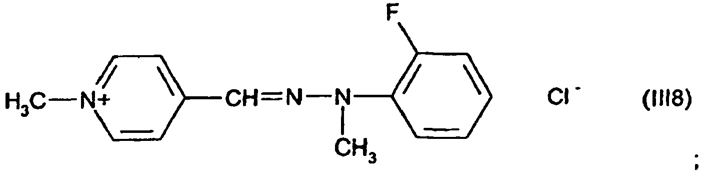

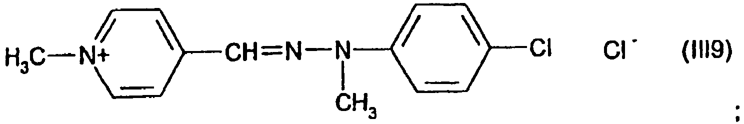

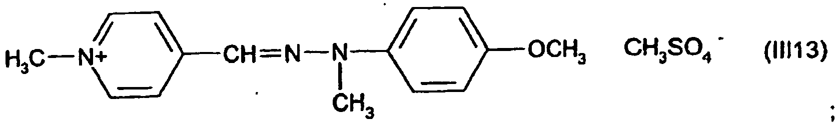

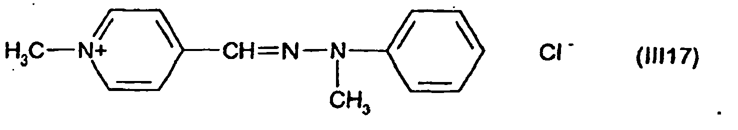

3. Composition selon une quelconque des revendications précédentes, **caractérisée par le fait que** le ou les colorants directs cationiques représentent de 0,00 à 10% en poids du poids total de la composition tinctoriale prête à l'emploi.

4. Composition selon la revendication 3, **caractérisée par le fait que** le ou les colorants directs cationiques représentent de 0,05 à 5 % en poids du poids total de la composition tinctoriale prête à l'emploi.

5. Composition selon l'une quelconque des revendications précédentes, **caractérisée par le fait que** le ou les colorants directs nitrés benzéniques sont choisis parmi les composés de formule (IV) suivante : 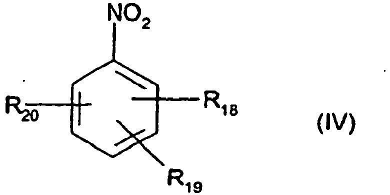 dans laquelle :
- R₁₈ représente un radical amino ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄) amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino,
- R₁₉ représente un atome d'hydrogène ; un radical amino ; hydroxyle ; alkyle en C₁-C₄ ; alcoxy en C₁-C₄ ; monohydroxyalkyle en C₁-C₄ ; polyhydroxyalkyle en C₂-C₄ ; monohydroxyalcoxy en C₁-C₄ ; polyhydroxyalcoxy en C₂-C₄ ; aminoalcoxy en C₁-C₄ ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄) amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino ;
- R₂₀ représente un atome d'hydrogène ou d'halogène, un radical, alkyle en C₁-C₄, ou un groupement nitro.

6. Composition selon la revendication 5, **caractérisée par le fait que** les colorants nitrés benzéniques de formule (IV) sont choisis parmi :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- le 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyéthyl)amino 5-méthoxy nitrobenzène,
- le 2-amino 5-β-hydroxyéthyloxy nitrobenzène.
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β-γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-β,γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthy)amino nitrobenzène,
- le 2-aminc 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino 5-amino nitrobenzène,
- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-(β-aminoéthyl)amino 4-hydroxy nitrobenzène,
- l'[hydroxy-2 N-(β-hydroxyéthyl)amino-3 nitro-6] benzyloxy]-2 éthylamine, et
- l'[hydroxy-2 N-(β-hydroxypropyl)amino-3 nitro-6] benzyloxy]-2 éthylamine.

7. Composition selon la revendication 6, **caractérisée par le fait que les** colorants nitrés benzéniques de formule (IV) sont choisis parmi :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(**-**β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

8. Composition selon l'une quelconque des revendications précédentes, **caractérisée par le fait que** le ou les colorants nitrés benzéniques représentent de 0,0005 à 15 % en poids de la composition tinctoriale prête à l'emploi.

9. Composition selon la revendication 8, **caractérisée par le fait que** le ou les colorants nitrés benzéniques représentent de 0,005 à 10 % en poids de la composition tinctoriale prête à l'emploi.

10. Composition selon l'une quelconque des revendications précédentes, **caractérisée par le fait qu'**elle renferme une ou plusieurs bases d'oxydation choisies parmi les paraphénylènediamines, les para-aminophénols, les orthophénylènediamines et les bases hétérocycliques et/ou un ou plusieurs coupleurs choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

11. Composition selon la revendication 10, **caractérisée par le fait que** la ou les bases d'oxydation représentent de 0,0005 à 12 % en poids du poids total de la composition tinctoriale prête à l'emploi et que le ou les coupleurs représentent de 0,0001 à 10 % en poids du poids total de la composition tinctoriale prête à l'emploi.

12. Composition selon la revendication 11, **caractérisée par le fait que** la ou les bases d'oxydation représentent de 0,005 à 8% en poids du poids total de la composition tinctoriale prête à l'emploi et que le ou les coupleurs représentent de 0,005 à 5 % en poids du poids total de la composition tinctoriale prête à l'emploi.

13. Composition selon l'une quelconque des revendications 10 à 12, **caractérisée par le fait que** les sels d'addition avec un acide sont choisis parmi les chlorhydrates, les bromhydrates, les sulfates, les tartrates, les lactates et les acétates.

14. Composition selon l'une quelconque des revendications 10 à 13, **caractérisée par le fait qu'**elle renferme au moins un agent oxydant.

15. Composition selon la revendication 14, **caractérisée par le fait que** l'agent oxydant est choisi parmi le peroxyde d'hydrogène, le peroxyde d'urée, les bromates de métaux alcalins, les persels tels que les perborates et persulfates, et les enzymes.

16. Composition selon la revendication 15, **caractérisée par le fait que** les enzymes sont choisies parmi les peroxydases et les oxydo-rédudases à deux électrons.

17. Composition selon la revendcation 16, **caractérisée par le fait que** les oxydo-réductases à deux électrons sont choisies parmi les pyranose oxydases, les glucose oxydases, les glycérol oxydases, les lactates oxydases, les pyruvate oxydases, et les uricases.

18. Composition selon la revendication 16 ou 17, **caractérisée par le fait que** l'oxydo-réductases à 2 électrons est choisie parmi les uricases d'origine animale, microbiologique ou biotechnologique.

19. Composition selon l'une quelconque des revendications 16 à 18 **caractérisée par le fait que** la ou les oxydo-réductases à 2 électrons représentent de 0,01 à 20 % en poids du poids total de la composition tinctoriale prête à l'emploi.

20. Composition selon la revendication 19 **caractérisée par le fait que** la ou les oxydo-réductases à 2 électrons représentent de 0,1 à 5 % en poids du poids total de la composition tinctoriale prête à l'emploi.

21. Composition selon l'une quelconque des revendications 18 à 20 **caractérisée par le fait qu'**elle renferme un donneur (ou substrat) pour ladite oxydo-réductase à 2 électrons, choisi parmi l'acide urique et ses sels.

22. Composition selon l'une quelconque des revendications précédentes, **caractérisée par le fait que** le milieu approprié pour la teinture est constitué par de l'eau ou par un mélange d'eau et d'au moins un solvant organique.

23. Composition selon l'une quelconque des revendications précédentes, **caractérisée par le fait qu'**elle présente un pH compris 5 et 11.

24. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

25. Procédé selon la revendication 24, **caractérisé par le fait qu'**il comporte une étape préliminaire consistant à stocker sous forme séparée, d'une part, une composition (A) comprenant, dans un milieu approprié pour la teinture, au moins un colorant direct cationique tel que défini à l'une quelconque des revendications 1 à 4, au moins un colorant direct nitrébenzénique etau moins une base d'oxydation et/ou au moins un coupleur et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

26. Dispositif à plusieurs compartiments ou "kit" de teinture, **caractérisé par le fait qu'**il comporte un premier compartiment renfermant la composition (A) telle que définie dans la revendication 25 et un second compartiment renfermant la composition (B) telle que définie dans la revendication 25.

## Patentansprüche

1. Gebrauchsfertige Zusammensetzung zum Färben von Keratinfasern und insbesondere menschlichen Keratinfasern, wie Haaren, **dadurch gekennzeichnet, dass** sie in einem zum Färben geeigneten Medium enthält:
- mindestens einen kationischen Direktfarbstoff, der ausgewählt ist unter:
b) Verbindungen der folgenden Formel (III): 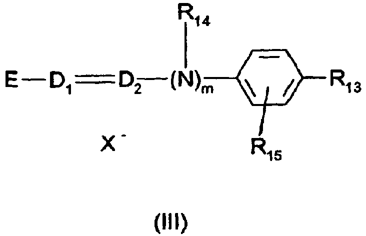 worin bedeuten:
R₁₃ ein Wasserstoffatom, C₁-₄-Alkoxy, ein Halogenatom, wie Brom, Chlor, Iod oder Fluor, oder eine Aminogruppe,
R₁₄ ein Wasserstoffatom, C₁₋₄-Alkyl, oder R₁₄ bildet mit einem Kohlenstoffatom des Benzolrings einen gegebenenfalls sauerstoffhaltigen Heterocyclus, der mit einer oder mehreren C₁₋₄-Alkylgruppen substituiert sein kann,
R₁₅ ein Wasserstoffatom oder ein Halogenatom, wie Brom, Chlor, Iod oder Fluor,
D₁ und D₂, die gleich oder verschieden sind, ein Stickstoffatom oder die Gruppe -CH,
m = 1,
X- ein Anion, vorzugsweise Chlorid, Methylsulfat und Acetat, E eine Gruppe, die unter den folgenden Strukturen E 1 bis E8 ausgewählt ist: 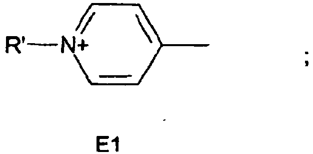 worin R' eine C₁₋₄-Alkylgruppe bedeutet;
- mindestens einen Direktfarbstoff vom nitrierten Benzoltyp.

2. Zusammensetzung nach Anspruch 1, **dadurch gekennzeichnet, dass** die kationischen Direktfarbstoffe der Formel (III) unter den Verbindungen der folgenden Strukturen ausgewählt sind:

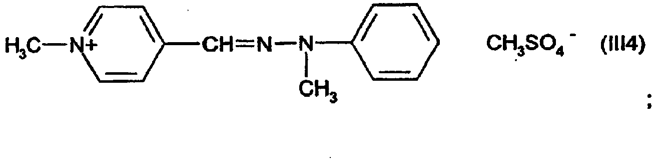

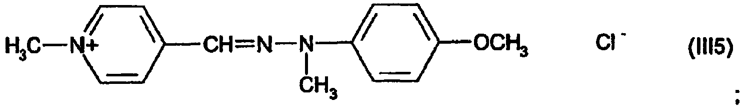

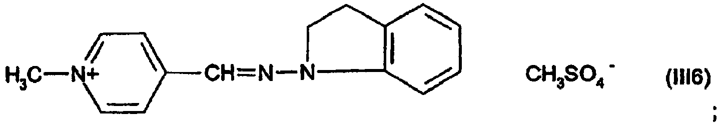

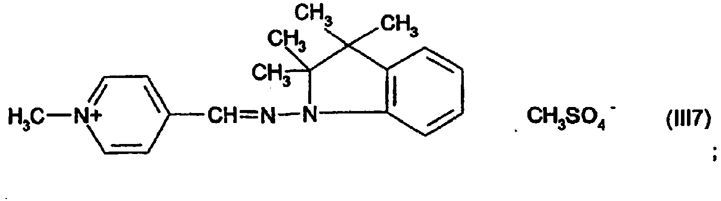

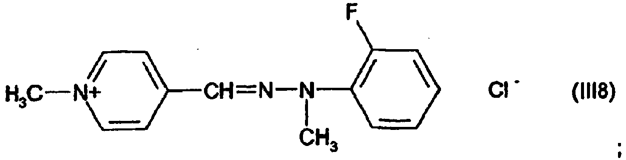

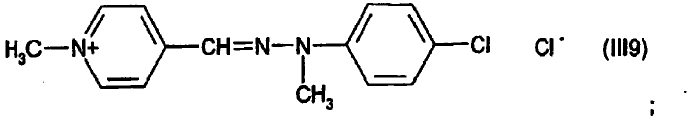

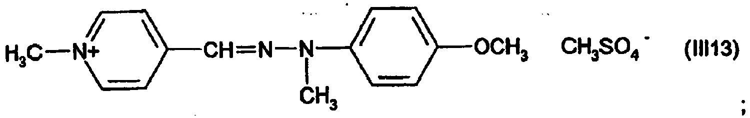

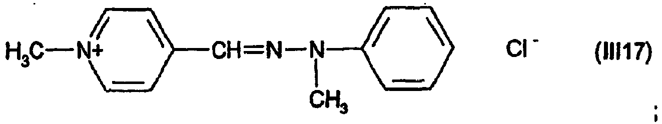

3. Zusammensetzung nach einem der vorhergehenden Ansprüche, **dadurch gekennzeichnet, dass** der oder die kationische(n) Direktfarbstoff(e) 0,001 bis 10 Gew.-% des Gesamtgewichts der gebrauchsfertigen Farbmittelzusammensetzung ausmachen.

4. Zusammensetzung nach Anspruch 3, **dadurch gekennzeichnet, dass** der oder die kationische(n) Direktfarbstoff(e) 0,05 bis 5 Gew.-% des Gesamtgewichts der gebrauchsfertigen Farbmittelzusammensetzung ausmachen.

5. Zusammensetzung nach einem der vorhergehenden Ansprüche, **dadurch gekennzeichnet, dass** der nitrierte Benzolfarbstoff oder die nitrierten Benzolfarbstoffe unter den Verbindungen der folgenden Formel (IV) ausgewählt sind: 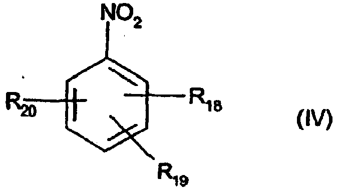 worin bedeuten:
- R₁₈ eine Aminogruppe; eine Aminogruppe, die mono- oder disubstituiert ist mit C₁₋₄-Alkyl, C₁-₄-Monohydroxyalkyl, C₂₋₄-Polyhydroxyalkyl, C₁-₄-Aminoalkyl, Mono-C₁₋₄-alkylamino-C₁₋₄-alkyl, Di-C₁₋₄-alkylamino-C₁₋₄-alkyl, C₁-₄-Ureidoalkyl, Aryl, einer Arylgruppe, deren Arylring mit einer oder mehreren Hydroxygruppen, Carboxygruppen, Aminogruppen oder Di-C₁-₄-alkylaminogruppen substituiert ist;
- R₁₉ ein Wasserstoffatom; eine Aminogruppe; Hydroxy; C₁₋₄-Alkyl, C₁₋₄-Alkoxy, C₁₋₄-Monohydroxyalkyl, C₂₋₄-Polyhydroxyalkyl, C₁₋₄-Monohydroxyalkoxy, C₂₋₄-Polyhydroxyalkoxy, C₁₋₄-Aminoalkoxy, eine Aminogruppe, die mono- oder disubstituiert ist mit C₁₋₄-Alkyl, C₁₋₄-Monohydroxyalkyl, C₂₋₄-Polyhydroxyalkyl, C₁₋₄-Aminoalkyl, Mono-C₁₋₄-alkylamino-C₁₋₄-alkyl, Di-C₁₋₄-alkylamino-C₁₋₄-alkyl, C₁₋₄-Ureidoalkyl, Aryl, einer Arylgruppe, deren Arylring mit einer oder mehreren Hydroxygruppen, Carboxygruppen, Aminogruppen oder Di-C₁₋₄-alkylaminogruppen substituiert ist;
- R₂₀ ein Wasserstoffatom; ein Halogenatom; C₁₋₄-Alkyl oder Nitro.

6. Zusammensetzung nach Anspruch 5, **dadurch gekennzeichnet, dass** die nitrierten Benzolfarbstoffe der Formel (IV) unter den folgenden Verbindungen ausgewählt sind:
- 2-Amino-4-methyl-5-N-(β-hydroxyethyl)amino-nitrobenzol,
- 4-N-(β-Ureidoethyl)amino-nitrobenzol,
- 4-(N-Ethyl-N-β-hydroxyethyl)amino-1-N-(β-hydroxyethyl)-amino-nitrobenzol,
- 2-N-(β-Hydroxyethyl)amino-5-methyl-nitrobenzol,
- 5-Chlor-3-N-(ethyl)amino-4-hydroxy-nitrobenzol,
- 5-Amino-3-chlor-4-hydroxy-nitrobenzol,
- 2-N-(γ-Hydroxypropyl)amino-5-N,N-bis(β-hydroxyethyl)amino-nitrobenzol,
- 5-Hydroxy-2-N-(γ-hydroxypropyl)amino-nitrobenzol,
- 1,3-Bis(β-hydroxyethyl)amino-4-chlor-6-nitrobenzol,
- 2,4-Diamino-nitrobenzol,
- 3,4-Diamino-nitrobenzol,
- 2,5-Diamino-nitrobenzol,
- 3-Amino-4-hydroxy-nitrobenzol,
- 4-Amino-3-hydroxy-nitrobenzol,
- 5-Amino-2-hydroxy-nitrobenzol,
- 2-Amino-5-hydroxy-nitrobenzol,
- 4-Amino-3-hydroxy-nitrobenzol,
- 5-Amino-2-hydroxy-nitrobenzol,
- 2-Amino-3-hydroxy-nitrobenzol,
- 2-Amino-5-N-(β-hydroxyethyl)amino-nitrobenzol,
- 2-Amino-5-N,N-bis(β-hydroxyethyl)amino-nitrobenzol,
- 2,5-N,N'-(β-Hydroxyethyl)amino-nitrobenzol,
- 2-N-(β-Hydroxyethyl)amino-5-N,N-bis(β-hydroxyethyl)amino-nitrobenzol,
- 2-Amino-5-N-(methyl)amino-nitrobenzol,
- 2-N-(Methyl)amino-5-N,N-bis(β-hydroxyethyl)amino-nitrobenzol,
- 2-N-(Methyl)amino-5-(N-methyl-N-β-hydroxyethyl)amino-nitrobenzol,
- 2,5-N,N'-(β-Hydroxyethyl)amino-nitrobenzol,
- 2-N-(β-Hydroxyethyl)amino-5-hydroxy-nitrobenzol,
- 3-Methoxy-4-N-(β-hydroxyethyl)amino-nitrobenzol,
- 2-N-(Methyl)amino-4-β-hydroxyethyloxy-nitrobenzol,
- 2-Amino-3-methyl-nitrobenzol,
- 2-N-(β-Hydroxyethyl)amino-5-amino-nitrobenzol,
- 2-Amino-4-chlor-5-N-(β-hydroxyethyl)amino-nitrobenzol,
- 2-Amino-4-methyl-5-N-(β-hydroxyethyl)amino-nitrobenzol,
- 2-Amino-4-methyl-5-N-(methyl)amino-nitrobenzol,
- 2-N-(β-Hydroxyethyl)amino-5-methoxy-nitrobenzol,
- 2-Amino-5-β-hydroxyethyloxy-nitrobenzol,
- 2-N-(β-Hydroxyethyl)amino-nitrobenzol,
- 3-Amino-4-N-(β-hydroxyethyl)amino-nitrobenzol,
- 3-β-Hydroxyethyloxy-4-N-(β-hydroxyethyl)amino-nitrobenzol,
- 2-N-(Methyl)amino-4-β,γ-dihydroxypropyloxy-nitrobenzol,
- 2-N-(β-Hydroxyethyl)amino-5-β-hydroxyethyloxy-nitrobenzol,
- 2-N-(β-Hydroxyethyl)amino-5-β,γ-dihydroxypropyloxy-nitrobenzol,
- 2-Hydroxy-4-N-(β-hydroxyethyl)amino-nitrobenzol,
- 2-N-(Methyl)amino-4-methyl-5-amino-nitrobenzol,
- 2-Amino-4-isopropyl-5-N-(methyl)amino-nitrobenzol,
- 2-N-(Methyl)amino-5-(N-methyl-N-β,γ-dihydroxypropyl)amino-nitrobenzol,
- 3-N-(β-Hydroxyethyl)amino-4-N-(β-hydroxyethyl)amino-nitrobenzol,
- 2-Amino-4-methyl-5-N-(β,γ-dihydroxypropyl)amino-nitrobenzol,
- 2-Amino-4-methyl-5-hydroxy-nitrobenzol,
- 2-N-(β-Hydroxyethyl)amino-4-N-(β-hydroxyethyl)amino-nitrobenzol,
- 2-Amino-5-N-(β-aminoethyl)amino-nitrobenzol,
- 2-N-(β-Aminoethyl)amino-5-methoxy-nitrobenzol,
- 2-N-(Methyl)amino-5-N-(β-aminoethyl)amino-nitrobenzol,
- 2-N-(β-Aminoethyl)amino-4-N,N-(dimethyl)amino-nitrobenzol,
- 3-Amino-4-N-(β-aminoethyl)amino-nitrobenzol,
- 2-Amino-4-methyl-5-N-(β-aminoethyl)amino-nitrobenzol,
- 2-N-(β-Aminoethyl)amino-5-N,N-bis(β-hydroxyethyl)amino-nitrobenzol,
- 3-β-Aminoethyloxy-4-amino-nitrobenzol,
- 2-N-(Methyl)amino-5-(N-δ-amino-*n*-butyl)amino-nitrobenzol,
- 2-N-(γ-Amino-*n*-propyl)amino-5-N,N-(dimethyl)amino-nitrobenzol,
- 3-Methoxy-4-N-(β-aminoethyl)amino-nitrobenzol,
- 2-N-(β-Aminoethyl)amino-5-amino-nitrobenzol,
- 2-Amino-4-chlor-5-N-(β-aminoethyl)amino-nitrobenzol,
- 2-N-(β-Aminoethyl)amino-4-methoxy-nitrobenzol,
- 2-N-(β-Aminoethyl)amino-nitrobenzol,
- 2-N-(β-Aminoethyl)amino-5-N-(β-aminoethyl)amino-nitrobenzol,
- 2-N-(β-Aminoethyl)amino-4-β-hydroxyethyloxy-nitrobenzol,
- 3-β-Hydroxyethyloxy-4-N-(β-aminoethyl)amino-nitrobenzol
- 2-Amino-5-aminoethyloxy-nitrobenzol,
- 3-Hydroxy-4-N-(β-aminoethyl)amino-nitrobenzol
- 2-N-(β-Aminoethyl)amino-5-β-hydroxyethyloxy-nitrobenzol,
- 2-N-(β-Aminoethyl)amino-4-hydroxy-nitrobenzol, 2-[2-Hydroxy-3-N-(β-hydroxyethyl)amino-6-nitro-benzyloxy]-ethylamin, und
- 2-[2-Hydroxy-3-N-(β-hydroxypropyl)amino-6-nitro-benzyloxy]-ethylamin.

7. Zusammensetzung nach Anspruch 6, **dadurch gekennzeichnet, dass** die nitrierten Benzolfarbstoffe der Formel (IV) unter den folgenden Verbindungen ausgewählt sind:
- 2-Amino-4-methyl-5-N-(β-hydroxyethyl)amino-nitrobenzol,
- 4-N-(β-Ureidoethyl)amino-nitrobenzol,
- 4-(N-Ethyl-N-β-hydroxyethyl)amino-1-N-(β-hydroxyethyl)-amino-nitrobenzol,
- 2-N-(β-Hydroxyethyl)amino-5-methyl-nitrobenzol,
- 5-Chlor-3-N-(ethyl)amino-4-hydroxy-nitrobenzol,
- 5-Amino-3-chlor-4-hydroxy-nitrobenzol,
- 2-N-(γ-Hydroxypropyl)amino-5-N,N-bis(β-hydroxyethyl)amino-nitrobenzol,
- 5-Hydroxy-2-N-(γ-hydroxypropyl)amino-nitrobenzol,
- 1,3-Bis(β-hydroxyethyl)amino-4-chlor-6-nitrobenzol,
- 3,4-Diamino-nitrobenzol,
- 2-Amino-5-hydroxy-nitrobenzol,
- 2-Amino-3-hydroxy-nitrobenzol,
- 2-Amino-5-N-(β-hydroxyethyl)amino-nitrobenzol,
- 2-Amino-5-N,N-bis(β-hydroxyethyl)amino-nitrobenzol,
- 2-N-(β-Hydroxyethyl)amino-5-N,N-bis(β-hydroxyethyl)amino-nitrobenzol,
- 2-N-(β-Hydroxyethyl)amino-5-hydroxy-nitrobenzol,
- 2-N-(β-Hydroxyethyl)amino-5-amino-nitrobenzol,
- 2-N-(β-Aminoethyl)amino-4-methoxy-nitrobenzol, und
- 2-N-(β-Aminoethyl)amino-5-β-hydroxyethyloxy-nitrobenzol.

8. Zusammensetzung nach einem der vorhergehenden Ansprüche, **dadurch gekennzeichnet, dass** der nitrierte Benzolfarbstoff oder die nitrierten Benzolfarbstoffe 0,0005 bis 15 Gew.-% der gebrauchsfertigen Farbmittelzusammensetzung ausmachen.

9. Zusammensetzung nach Anspruch 8, **dadurch gekennzeichnet, dass** der nitrierte Benzolfarbstoff oder die nitrierten Benzolfarbstoffe 0,005 bis 10 Gew.-% der gebrauchsfertigen Farbmittelzusammensetzung ausmachen.

10. Zusammensetzung nach einem der vorhergehenden Ansprüche, **dadurch gekennzeichnet, dass** sie eine oder mehrere Oxidationsbasen, die unter den p-Phenylendiaminen, p-Aminophenolen, o-Phenylendiaminen und heterocyclischen Basen ausgewählt sind, und/oder einen oder mehrere Kuppler enthält, die unter den m-Phenylendiaminen, m-Aminophenolen, m-Dihydroxybenzolen, heterocyclischen Kupplern, wie Indolderivaten, Indolinderivaten, Benzimidazolderivaten, Benzomorpholinderivaten, Sesamolderivaten, Pyridinderivaten, Pyrimidinderivaten und Pyrazolderivaten, und deren Additionssalzen mit einer Säure ausgewählt sind.

11. Zusammensetzung nach Anspruch 10, **dadurch gekennzeichnet, dass** die Oxidationsbase(n) 0,0005 bis 12 Gew.-% des Gesamtgewichts der gebrauchsfertigen Farbmittelzusammensetzung und der oder die Kuppler 0,0001 bis 10 Gew.-% des Gesamtgewichts der gebrauchsfertigen Farbmittelzusammensetzung ausmachen.

12. Zusammensetzung nach Anspruch 11, **dadurch gekennzeichnet, dass** die Oxidationsbase(n) 0,005 bis 8 Gew.-% des Gesamtgewichts der gebrauchsfertigen Farbmittelzusammensetzung und der oder die Kuppler 0,005 bis 5 Gew.-% des Gesamtgewichts der gebrauchsfertigen Farbmittelzusammensetzung ausmachen.

13. Zusammensetzung nach einem der Ansprüche 10 bis 12, **dadurch gekennzeichnet, dass** die Additionssalze mit einer Säure unter den Hydrochloriden, Hydrobromiden, Sulfaten, Tartraten, Lactaten und Acetaten ausgewählt sind.

14. Zusammensetzung nach einem der Ansprüche 10 bis 13, **dadurch gekennzeichnet, dass** sie mindestens ein Oxidationsmittel enthält.

15. Zusammensetzung nach Anspruch 14, **dadurch gekennzeichnet, dass** das Oxidationsmittel unter Wasserstoffperoxid, Harnstoff peroxid, Alkalimetallbromaten, Salzen von Persäuren, wie Perboraten und Persulfaten, und Enzymen ausgewählt ist.

16. Zusammensetzung nach Anspruch 15, **dadurch gekennzeichnet, dass** die Enzyme unter den Peroxidasen und den Oxidoreduktasen (zwei Elektronen) ausgewählt sind.

17. Zusammensetzung nach Anspruch 16, **dadurch gekennzeichnet, dass** die Oxidoreduktasen (zwei Elektronen) unter den Pyranose-Oxidasen, Glucose-Oxidasen, Glycerin-Oxidasen, Lactat-Oxidasen, Pyruvat-Oxidasen und Uricasen ausgewählt sind.

18. Zusammensetzung nach Anspruch 16 oder 17, **dadurch gekennzeichnet, dass** die Oxidoreduktasen (2 Elektronen) unter den Uricasen tierischer, mikrobiologischer oder biotechnologischer Herkunft ausgewählt sind.

19. Zusammensetzung nach einem der Ansprüche 16 bis 18, **dadurch gekennzeichnet, dass** die Oxidoreduktase(n) (2 Elektronen) 0,01 bis 20 Gew.-% des Gesamtgewichts der gebrauchsfertigen Farbmittelzusammensetzung ausmachen.

20. Zusammensetzung nach Anspruch 19, **dadurch gekennzeichnet, dass** die Oxidoreduktase(n) (2 Elektronen) 0,1 bis 5 Gew.-% des Gesamtgewichts der gebrauchsfertigen Farbmittelzusammensetzung ausmachen.

21. Zusammensetzung nach einem der Ansprüche 18 bis 20, **dadurch gekennzeichnet, dass** der Donor (oder das Substrat) für die Oxidoreduktase (2 Elektronen) unter Harnsäure und ihren Salzen ausgewählt ist.

22. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

23. Zusammensetzung nach einem der vorhergehenden Ansprüche, **dadurch gekennzeichnet, dass** sie einen pH-Wert im Bereich von 5 bis 11 aufweist.

24. Verfahren zum Färben von Keratinfasern und insbesondere menschlichen Keratinfasern, wie dem Haar, **dadurch gekennzeichnet, dass** auf die Fasern mindestens eine gebrauchsfertige Farbmittelzusammensetzung nach einem der vorhergehenden Ansprüche während einer Zeitspanne, die zur Bildung der gewünschten Färbung ausreichend ist, aufgebracht wird.

25. Verfahren nach Anspruch 24, **dadurch gekennzeichnet, dass** es einen vorbereitenden Schritt umfasst, der darin besteht, einerseits eine Zusammensetzung (A), die in einem zum Färben geeigneten Medium mindestens einen in den Ansprüchen 1 bis 4 definierten Direktfarbstoff, mindestens einen Direktfarbstoff vom nitrierten Benzoltyp und mindestens eine Oxidationsbase und/oder mindestens einen Kuppler enthält, und andererseits eine Zusammensetzung (B) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird.

26. Vorrichtung mit mehreren Abteilungen oder "Kit" zum Färben mit mehreren Abteilungen, **dadurch gekennzeichnet, dass** eine erste Abteilung die Zusammensetzung (A) nach Anspruch 25 und eine andere Abteilung die Zusammensetzung (B) nach Anspruch 25 enthält.

## Claims

1. Ready-to-use composition for dyeing keratin fibres, and in particular human keratin fibres such as the hair, **characterized in that** it comprises, in a medium which is suitable for dyeing:
- at least one cationic direct dye chosen from:
b) the compounds of formula (III) below: 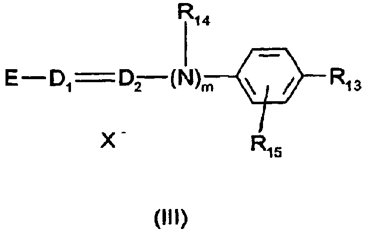 in which:
R₁₃ represents a hydrogen atom, a C₁-C₄ alkoxy radical, a halogen atom such as bromine, chlorine, iodine or fluorine or an amino radical,
R₁₄ represents a hydrogen atom or a C₁-C₄ alkyl radical or forms, with a carbon atom of the benzene ring, a heterocycle which is optionally oxygenated and/or substituted by one or more C₁-C₄ alkyl groups,
R₁₅ represents a hydrogen or halogen atom such as bromine, chlorine, iodine or fluorine,
D₁ and D₂, which are identical or different, represent a nitrogen atom or the group -CH,
m=1,
X⁻ represents an anion preferably chosen from chloride, methyl sulphate and acetate,
E represents a group chosen from structures below: 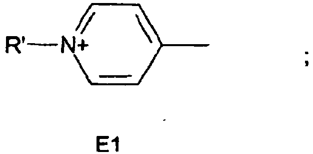
in which R' represents a C₁-C₄ alkyl radical;
- and at least one nitrobenzene direct dye.

2. Composition according to Claim 1, **characterized in that** the cationic direct dyes of formula (III) are chosen from the compounds corresponding to structures below:

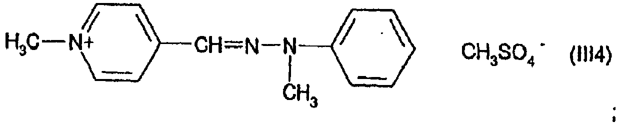

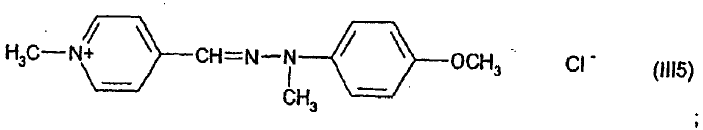

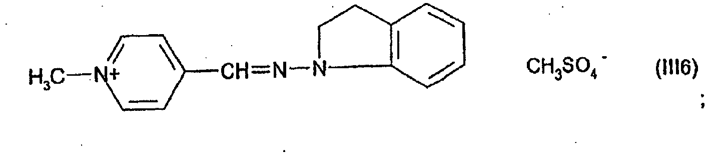

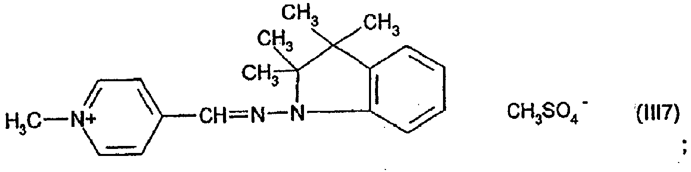

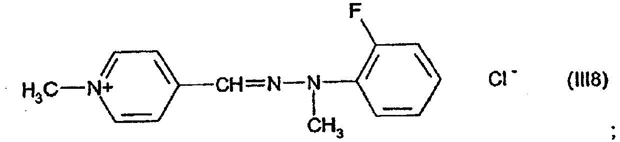

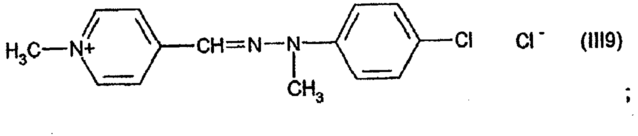

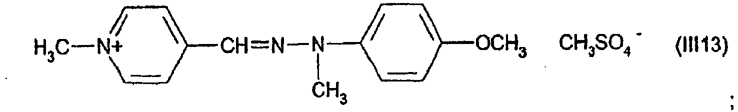

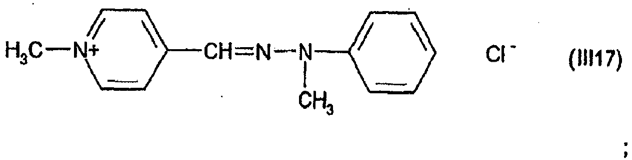

3. Composition according to any one of the preceding claims, **characterized in that** the cationic direct dye(s) represent(s) from 0.001 to 10% by weight relative to the total weight of the ready-to-use dye composition.

4. Composition according to Claim 3, **characterized in that** the cationic direct dye(s) represent(s) from 0.05 to 5% by weight relative to the total weight of the ready-to-use dye composition.

5. Composition according to any one of the preceding claims, **characterized in that** the nitrobenzene direct dye(s) is (are) chosen from the compounds of formula (IV) below: 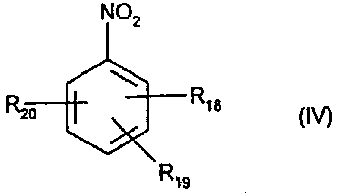 in which:
- R₁₈ represents an amino radical; an amino radical monosubstituted or disubstituted with a C₁-C₄ alkyl, C₁-C₄ monohydroxyalkyl, C₂-C₄ polyhydroxyalkyl, C₁-C₄ aminoalkyl, mono(C₁-C₄) alkylamino (C₁-C₄) alkyl, di (C₁-C₄) alkylamino (C₁-C₄) alkyl or ureido(C₁-C₄)alkyl or aryl radical or an aryl radical in which the aryl ring is substituted with one or more hydroxyl, carboxyl, amino or di (C₁-C₄)alkylamino radicals,
- R₁₉ represents a hydrogen atom; an amino radical; hydroxyl radical; C₁-C₄ alkyl radical; C₁-C₄ alkoxy radical; C₁-C₄ monohydroxyalkyl radical; C₂-C₄ polyhydroxyalkyl radical; C₁-C₄ monohydroxyalkoxy radical; C₂-C₄ polyhydroxyalkoxy radical; C₁-C₄ aminoalkoxy radical; an amino radical monosubstituted or disubstituted with a C₁-C₄ alkyl, C₁-C₄ monohydroxyalkyl, C₂-C₄ polyhydroxyalkyl, C₁-C₄ aminoalkyl, mono (C₁-C₄) - alkylamino (C₁-C₄) alkyl, di (C₁-C₄) alkylamino (C₁-C₄) alkyl or ureido (C₁-C₄) alkyl or aryl radical or an aryl radical in which the aryl ring is substituted with one or more hydroxyl, carboxyl, amino or di(C₁-C₄)alkylamino radicals;
- R₂₀ represents a hydrogen or halogen atom, a C₁-C₄ alkyl radical or a nitro group.

6. Composition according to Claim 5, **characterized in that** the nitrobenzene dyes of formula (IV) are chosen from:
- 2-amino-4-methyl-5-N-(β-hydroxyethyl)aminonitro-benzene,
- 4-N-(β-ureidoethyl)aminonitrobenzene,
- 4-(N-ethyl-N-β-hydroxyethyl)amino-1-N-(β-hydroxyethyl)aminonitrobenzene,
- 2-N-(β-hydroxyethyl)amino-5-methylnitrobenzene,
- 5-chloro-3-N-(ethyl)amino-4-hydroxynitrobenzene,
- 5-amino-3-chloro-4-hydroxynitrobenzene,
- 2-N-(γ-hydroxypropyl)amino-5-N,N-bis(β-hydroxyethyl)-aminonitrobenzene,
- 5-hydroxy-2-N-(γ-hydroxypropyl)aminonitrobenzene,
- 1,3-bis(β-hydroxyethyl)amino-4-chloro-6-nitrobenzene,
- 2,4-diaminonitrobenzene,
- 3,4-diaminonitrobenzene,
- 2,5-diaminonitrobenzene,
- 3-amino-4-hydroxynitrobenzene,
- 4-amino-3-hydroxynitrobenzene,
- 5-amino-2-hydroxynitrobenzene,
- 2-amino-5-hydroxynitrobenzene,
- 4-amino-3-hydroxynitrobenzene,
- 5-amino-2-hydroxynitrobenzene,
- 2-amino-3-hydroxynitrobenzene,
- 2-amino-5-N-(β-hydroxyethyl)aminonitrobenzene,
- 2-amino-5-N,N-bis(3-hydroxyethyl)aminonitrobenzene,
- 2,5-N,N'-(β-hydroxyethyl)aminonitrobenzene,
- 2-N-(β-hydroxyethyl)amino-5-N,N-bis(β-hydroxyethyl)-aminonitrobenzene,
- 2-amino-5-N-(methyl)aminonitrobenzene,
- 2-N-(methyl)amino-5-N,N-bis(β-hydroxyethyl)aminonitrobenzene,
- 2-N-(methyl)amino-5-(N-methyl-N-β-hydroxyethyl)aminonitrobenzene,
- 2,5-N,N'-(β-hydroxyethyl)aminonitrobenzene,
- 2-N-(β-hydroxyethyl)amino-5-hydroxynitrobenzene,
- 3-methoxy-4-N-(β-hydroxyethyl)aminonitrobenzene,
- 2-N-(methyl)amino-4-β-hydroxyethyloxynitrobenzene,
- 2-amino-3-methylnitrobenzene,
- 2-N-(β-hydroxyethyl)amino-5-aminonitrobenzene,
- 2-amino-4-chloro-5-N-(β-hydroxyethyl)aminonitrobenzene,
- 2-amino-4-methyl-5-N-(β-hydroxyethyl)aminonitrobenzene,
- 2-amino-4-methyl-5-N-(methyl)aminonitrobenzene,
- 2-N-(β-hydroxyethyl)amino-5-methoxynitrobenzene,
- 2-amino-5-β-hydroxyethyloxynitrobenzene,
- 2-N-(β-hydroxyethyl)aminonitrobenzene,
- 3-amino-4-N-(β-hydroxyethyl)aminonitrobenzene,
- 3-β-hydroxyethyloxy-4-N-(β-hydroxyethyl)aminonitrobenzene,
- 2-N-(methyl)amino-4-β,γ-dihydroxypropyloxynitrobenzene,
- 2-N-(β-hydroxyethyl)amino-5-β-hydroxyethyloxynitrobenzene,
- 2-N-(β-hydroxyethyl)amino-5-β,γ-dihydroxypropyloxynitrobenzene,
- 2-hydroxy-4-N-(β-hydroxyethyl)aminonitrobenzene,
- 2-N-(methyl)amino-4-methyl-5-aminonitrobenzene,
- 2-amino-4-isopropyl-5-N-(methyl)aminonitrobenzene,
- 2-N-(methyl)amino-5-(N-methyl-N-β,γ-dihydroxypropyl)-aminonitrobenzene,
- 3-N-(β-hydroxyethyl)amino-4-N-(β-hydroxyethyl)aminonitrobenzene,
- 2-amino-4-methyl-5-N-(β,γ-dihydroxypropyl) aminonitrobenzene,
- 2-amino-4-methyl-5-hydroxynitrobenzene,
- 2-N-(β-hydroxyethyl) amino-4-N-(β-hydroxyethyl) aminonitrobenzene,
- 2-amino-5-N-(β-aminoethyl)aminonitrobenzene,
- 2-N-(β-aminoethyl)amino-5-methoxynitrobenzene,
- 2-N-(methyl)amino-5-N-(β-aminoethyl)aminonitrobenzene,
- 2-N-(β-aminoethyl)amino-4-N,N-(dimethyl)aminonitrobenzene,
- 3-amino-4-N-(β-aminoethyl)aminonitrobenzene,
- 2-amino-4-methyl-5-N-(β-aminoethyl)aminonitrobenzene,
- 2-N-(β-aminoethyl)amino-5-N,N-bis(β-hydroxyethyl)-aminonitrobenzene,
- 3-β-aminoethyloxy-4-aminonitrobenzene,
- 2-N-(methyl)amino-5-(N-δ-amino-n-butyl)aminonitrobenzene,
- 2-N-(γ-amino-n-propyl)amino-5-N,N-(dimethyl)aminonitrobenzene,
- 3-methoxy-4-N-(β-aminoethyl)aminonitrobenzene,
- 2-N-(β-aminoethyl)amino-5-aminonitrobenzene,
- 2-amino-4-chloro-5-N-(β-aminoethyl)aminonitrobenzene,
- 2-N-(β-aminoethyl)amino-4-methoxynitrobenzene,
- 2-N-(β-aminoethyl)aminonitrobenzene,
- 2-N-(β-aminoethyl)amino-5-N-(β-aminoethyl)aminonitrobenzene,
- 2-N-(β-aminoethyl)amino-4-β-hydroxyethyloxynitrobenzene,
- 3-β-hydroxyethyloxy-4-N-(β-aminoethyl)aminonitrobenzene,
- 2-amino-5-aminoethyloxynitrobenzene,
- 3-hydroxy-4-N-(β-aminoethyl)aminonitrobenzene,
- 2-N-(β-aminoethyl)amino-5-β-hydroxyethyloxynitrobenzene,
- 2-N-(β-aminoethyl)amino-4-hydroxynitrobenzene,
- 2-{[2-hydroxy-3-N-(β-hydroxyethyl)amino-6-nitro]benzyloxy}ethylamine, and
- 2-{[2-hydroxy-3-N-(β-hydroxypropyl)amino-6-nitro]benzyloxy}ethylamine.

7. Composition according to Claim 6, **characterized in that** the nitrobenzene dyes of formula (IV) are chosen from:
- 2-amino-4-methyl-5-N-(β-hydroxyethyl)aminonitrobenzene,
- 4-N-(β-ureidoethyl)aminonitrobenzene,
- 4-(N-ethyl-N-β-hydroxyethyl)amino-1-N-(β-hydroxyethyl)aminonitrobenzene,
- 2-N-(β-hydroxyethyl)amino-5-methylnitrobenzene,
- 5-chloro-3-N-(ethyl)amino-4-hydroxynitrobenzene,
- 5-amino-3-chloro-4-hydroxynitrobenzene,
- 2-N-(γ-hydroxypropyl)amino-5-N,N-bis(β-hydroxyethyl)-aminonitrobenzene,
- 5-hydroxy-2-N-(γ-hydroxypropyl)aminonitrobenzene,
- 1,3-bis(β-hydroxyethyl)amino-4-chloro-6-nitrobenzene,
- 3,4-diaminonitrobenzene,
- 2-amino-5-hydroxynitrobenzene,
- 2-amino-3-hydroxynitrobenzene,
- 2-amino-5-N-(β-hydroxyethyl)aminonitrobenzene,
- 2-amino-5-N,N-bis(β-hydroxyethyl)aminonitrobenzene,
- 2-N-(β-hydroxyethyl)amino-5-N,N-bis(β-hydroxyethyl)-aminonitrobenzene,
- 2-N-(β-hydroxyethyl)amino-5-hydroxynitrobenzene,
- 2-N-(β-hydroxyethyl)amino-5-aminonitrobenzene,
- 2-N-(β-aminoethyl)amino-4-methoxynitrobenzene, and
- 2-N-(β-aminoethyl)amino-5-β-hydroxyethyloxynitrobenzene.

8. Composition according to any one of the preceding claims, **characterized in that** the nitrobenzene dye(s) represent(s) from 0.0005 to 15% by weight of the ready-to-use dye composition.

9. Composition according to Claim 8, **characterized in that** the nitrobenzene dye(s) represent(s) from 0.005 to 10% by weight of the ready-to-use dye composition.

10. Composition according to any one of the preceding claims, **characterized in that** it contains one or more oxidation bases chosen from para-phenylenediamines, para-aminophenols, ortho-phenylenediamines and heterocyclic bases and/or one or more couplers chosen from meta-phenylenediamines, meta-aminophenols, meta-diphenols, heterocyclic couplers such as, for example, indole derivatives, indoline derivatives, benzimidazole derivatives, benzomorpholine derivatives, sesamol derivatives, pyridine, pyrimidine and pyrazole derivatives, and the addition salts thereof with an acid.

11. Composition according to Claim 12, **characterized in that** the oxidation base(s) represent(s) from 0.0005 to 12% by weight relative to the total weight of the ready-to-use dye composition and **in that** the coupler(s) represent(s) from 0.0001 to 10% by weight relative to the total weight of the ready-to-use dye composition.

12. Composition according to Claim 11, **characterized in that** the oxidation base(s) represent(s) from 0.005 to 8% by weight relative to the total weight of the ready-to-use dye composition and **in that** the coupler(s) represent(s) from 0.005 to 5% by weight relative to the total weight of the ready-to-use dye composition.

13. Composition according to any one of Claims 10 to 12, **characterized in that** the addition salts with an acid are chosen from the hydrochlorides, hydrobromides, sulphates, tartrates, lactates and acetates.

14. Composition according to any one of Claims 10 to 13, **characterized in that** it contains at least one oxidizing agent.

15. Composition according to Claim 14, **characterized in that** the oxidizing agent is chosen from hydrogen peroxide, urea peroxide, alkali metal bromates, persalts such as perborates and persulphates, and enzymes.

16. Composition according to Claim 15, **characterized in that** the enzymes are chosen from peroxidases and two-electron oxidoreductases.

17. Composition according to Claim 16, **characterized in that** the two-electron oxidoreductases are chosen from pyranose oxidases, glucose oxidases, glycerol oxidases, lactate oxidases, pyruvate oxidases and uricases.

18. Composition according to Claim 16 or 17, **characterized in that** the 2-electron oxidoreductase is chosen from uricases of animal, microbiological or biotechnological origin.

19. Composition according to any one of Claims 16 to 18, **characterized in that** the 2-electron oxidoreductase(s) represent(s) from 0.01 to 20% by weight relative to the total weight of the ready-to-use dye composition.

20. Composition according to Claim 19, **characterized in that** the 2-electron oxidoreductase(s) represent(s) from 0.1 to 5% by weight relative to the total weight of the ready-to-use dye composition.

21. Composition according to any one of Claims 18 to 20, **characterized in that** it contains a donor (or substrate) for the said 2-electron oxidoreductase, chosen from uric acid and its salts.

22. Composition according to any one of the preceding claims, **characterized in that** the medium which is suitable for dyeing consists of water or a mixture of water and at least one organic solvent.

23. Composition according to any one of the preceding claims, **characterized in that** it has a pH of between 5 and 11.

24. Process for dyeing keratin fibres, and in particular human keratin fibres such as the hair, **characterized in that** at least one ready-to-use dye composition as defined in any one of the preceding claims is applied to the said fibres for a period which is sufficient to develop the desired coloration.

25. Process according to Claim 24, **characterized in that** it includes a preliminary step which consists in separately storing, on the one hand, a composition (A) comprising, in a medium which is suitable for dyeing, at least one cationic direct dye as defined in any one of Claims 1 to 4, at least one nitrobenzene direct dye and at least one oxidation base and/or at least one coupler, and, on the other hand, a composition (B) containing, in a medium which is suitable for dyeing, at least one oxidizing agent, and then in mixing them together at the time of use, before applying this mixture to the keratin fibres.

26. Multi-compartment dyeing device or kit, **characterized in that** it includes a first compartment containing composition (A) as defined in Claim 25 and a second compartment containing composition (B) as defined in Claim 25.
